(19) **Europäisches Patentamt / European Patent Office / Office européen des brevets**

(11) **EP 1 877 831 B1**

(12) **FASCICULE DE BREVET EUROPEEN**

(45) Date de publication et mention
de la délivrance du brevet:
**14.12.2011 Bulletin 2011/50**

(51) Int Cl.:
***G01T 1/161*** *(2006.01)*

(21) Numéro de dépôt: **06755468.3**

(22) Date de dépôt: **02.05.2006**

(86) Numéro de dépôt international:
**PCT/FR2006/000983**

(87) Numéro de publication internationale:
**WO 2006/117475 (09.11.2006 Gazette 2006/45)**

(54) **GAMMA CAMERA POUR LA LOCALISATION DES GANGLIONS SENTINELLES**

GAMMAKAMERA ZUR LOKALISIERUNG VON SENTINEL-LYMPHKNOTEN

GAMMA CAMERA FOR LOCALIZING SENTINEL NODES

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **02.05.2005 FR 0504448**

(43) Date de publication de la demande:
**16.01.2008 Bulletin 2008/03**

(73) Titulaires:
- **CENTRE NATIONAL DE LA RECHERCHE SCIENTIFIQUE (CNRS)**
  **75794 Paris Cedex 16 (FR)**
- **Université de Strasbourg**
  **67000 Strasbourg (FR)**

(72) Inventeurs:
- **GUYONNET, Jean-Louis**
  **F-67120 Soultz-Les-Bains (FR)**
- **BRASSE, David**
  **F-67117 Fessenheim-le-Bas (FR)**
- **MATHELIN, Carole**
  **F-67000 Strasbourg (FR)**
- **STAUB, Denis**
  **F-67200 Strasbourg (FR)**

(74) Mandataire: **Cabinet Plasseraud et al**
**52, rue de la Victoire**
**75440 Paris Cedex 09 (FR)**

(56) Documents cités:
WO-A-96/37791          US-A1- 2003 081 716
US-B1- 6 242 744       US-B2- 6 671 541

- ZIEGLER S I ET AL: "Characteristics of the new YAlO3:Ce compared with BGO and GSO" IEEE TRANSACTIONS ON NUCLEAR SCIENCE USA, vol. 40, no. 2, avril 1993 (1993-04), pages 194-197, XP002367071 ISSN: 0018-9499
- GORIN A ET AL: "Fundamental properties of YAP Imager" NUCLEAR INSTRUMENTS & METHODS IN PHYSICS RESEARCH, SECTION - A: ACCELERATORS, SPECTROMETERS, DETECTORS AND ASSOCIATED EQUIPMENT, ELSEVIER, AMSTERDAM, NL, vol. 510, no. 1-2, 1 septembre 2003 (2003-09-01), pages 76-82, XP004450947 ISSN: 0168-9002
- KNOLL, G. F.: "Radiation detection and measurement" 1989, JOHN WILEY & SONS , XP002367054 page 240 - page 241 page 254, alinéa D - page 255; figure 9.2
- BERTOLUCCI E ET AL: "Preliminary test of an imaging probe for nuclear medicine using hybrid pixel detectors" NUCLEAR INSTRUMENTS & METHODS IN PHYSICS RESEARCH, SECTION - A: ACCELERATORS, SPECTROMETERS, DETECTORS AND ASSOCIATED EQUIPMENT, ELSEVIER, AMSTERDAM, NL, vol. 487, no. 1-2, 11 juillet 2002 (2002-07-11), pages 193-201, XP004369022 ISSN: 0168-9002
- NOTARISTEFANI ET AL: "A YAP Camera 40 x 40 mm2 with fast readout electronics", IEEE TRANSACTIONS ON NUCLEAR SCIENCE, vol. 45, no. 5, October 1998 (1998-10), pages 2302-2308,

**Description**

La présente invention concerne le domaine des techniques médicales pour la localisation de tumeurs ou analogues, plus particulièrement les dispositifs et caméras destinés à être utilisés sur le corps humain. Elle a pour objet un dispositif de localisation opératoire de tissus marqués, notamment par des isotopes radioactifs afin de permettre la détection et l'identification des ganglions lymphatiques.

La localisation de tissus marqués à l'aide de marqueurs radioactifs ou isotopes émettant des rayonnements γ est connue pour l'identification suivie de l'excision de ganglions lymphatiques dits ganglions sentinelles. Les ganglions axillaires sont, par exemple, d'excellents indicateurs du pronostic et du traitement à mettre en oeuvre dans le cadre des cancers du sein.

La technique du ganglion sentinelle en développement depuis quelques années a pour objectif l'amélioration du diagnostic du stade évolutif de nombreux cancers lymphophiles comme le cancer du sein, de la prostate, le mélanome ou d'autres cancers. L'envahissement métastatique de ces cancers s'effectue via le système lymphatique, le ganglion sentinelle étant le premier ganglion à recevoir le flux lymphatique provenant de la tumeur. Après injection de radiocolloïdes (sulfure de rhénium ou albumine par exemple marqué avec du technétium $^{99m}$Tc) au niveau de la tumeur ou dans d'autres régions appropriées, le marquage des ganglions sentinelles résulte de la rétention de ces molécules par les cellules macrophages des ganglions. Une localisation précise des ganglions sentinelles permet un prélèvement de ces derniers, pour déterminer leur état d'envahissement par des cellules cancéreuses. Une analyse anatomopathologique extemporanée de ces ganglions permettra de déterminer s'il convient ou non de pratiquer une exérèse complète de la chaîne ganglionnaire.

L'analyse du système de drainage lymphatique permet donc d'apprécier de manière très précise et fiable le degré d'extension du cancer et de déterminer l'étendue d'une éventuelle exérèse (mutilante) de la chaîne ganglionnaire, suivie de la mise en place d'une thérapie complémentaire, ou au contraire, dans le cas d'un résultat négatif, d'une chirurgie ganglionnaire conservatrice (lorsque le(s) ganglion(s) radioactif(s) sont indemnes de cellules cancéreuses, le curage ganglionnaire traditionnel est évité). Il s'agit en effet aujourd'hui d'éviter une exérèse systématique de la chaîne ganglionnaire, cette exérèse étant non seulement inutile lorsque les ganglions sont indemnes de cellules cancéreuses, mais aussi délétère car elle entraîne des conséquences handicapantes dans la vie de tous les jours de la patiente (troubles de la mobilité, troubles de la sensibilité, lymphoedème...).

L'intervention chirurgicale est généralement précédée d'une lymphoscintigraphie réalisée dans un Service de Médecine Nucléaire et permettant de localiser et dénombrer les ganglions sentinelles.

Les techniques actuelles visent une sélectivité accrue de la détection afin de localiser avec précision le ganglion sentinelle. On connaît des sondes qui permettent au praticien de localiser avec précision le ganglion sentinelle au cours de l'opération. Un tel dispositif, connu de la demande française FR 2 823 092, utilise la transformation du rayonnement radioactif en photons de scintillation par un cristal scintillant, cristal couplé à un photomultiplicateur. Ce dispositif ne permet cependant qu'une recherche un par un des ganglions sentinelles. On connaît également des gamma caméras permettant une localisation 2D de plusieurs ganglions sentinelles et utilisant des cristaux d'iodure de sodium ou d'iodure de césium couplés à un ensemble de photomultiplicateurs. Ces dispositifs sont généralement très volumineux et peu maniables par le praticien, notamment au cours de l'opération.

Le document US 6 671 541 décrit un dispositif d'imagerie non invasif pour évaluer la santé cardiaque d'un patient. Ce dispositif utilise au moins un détecteur gamma et un circuit électronique qui traite les signaux et les dirige vers un système d'acquisition rapide. Ce dispositif est volumineux. Le document US6242744 decrit un dispositif selon la preambule de la revendication 1.

Le document WO 96/37791 décrit un dispositif d'analyse scintigraphique, et plus particulièrement un dispositif de mammographie avec une résolution spatiale submillimétrique. Une tête sensible comprend un collimateur, des cristaux scintillateurs placés sur ledit collimateur, des photomultiplicateurs et un préamplificateur.

Le document US 2003/081716 décrit une sonde d'imagerie compacte, portative, à semi-conducteur et à haute sensibilité. Ce dispositif est utilisé pour la détection et la localisation de ganglion sentinelle lymphatique.

Le but de la présente invention est de proposer un dispositif permettant de détecter avec une efficacité maximale le rayonnement γ émis par un traceur radioactif (notamment un radiocolloïde) concentré dans un ou plusieurs ganglions sentinelles pour repérer ces derniers dans l'ensemble de la chaîne ganglionnaire visée.

A cet effet, la présente invention a pour objet un dispositif pour la localisation opératoire de tissus marqués selon la revendication 1 et comprenant :

- un collimateur apte à sélectionner un rayonnement radioactif émis par des tissus marqués par au moins un marqueur radioactif dans au moins une direction définie, ledit collimateur étant attaché à un blindage extérieur,
- au moins un cristal scintillant pour transformer en photons de scintillation ledit rayonnement radioactif, ledit cristal scintillant étant entouré par le blindage pour le protéger du bruit radioactif ambiant ; le cristal scintillant étant un cristal inorganique préférablement non hygroscopique, présentant un rendement en photons de scintillation superieur à 9000 photons /Mev,

- des moyens de transformation multivoies desdits photons de scintillation en signaux électriques de détection, lesdits signaux électriques étant fonction notamment des voies ayant été touchées par lesdits photons de scintillation, lesdits moyens de transformation multivoies étant insérés dans le blindage ;
- des moyens de traitement électronique (40) des signaux électriques produits par les moyens de transformation multivoies, lesdits moyens de traitement étant placés à proximité immédiate desdits moyens de transformation multivoies pour que le dispositif ait un faible encombrement et soit maniable, et comprenant au moins un circuit intégré comprenant un étage d'intégration desdits signaux électriques adapté pour fournir une mesure de la charge électrique desdits signaux électriques en une durée inférieure à 200 ns .

Ainsi ce dispositif assure une sélectivité accrue permettant la production grâce aux moyens de traitement d'une image 2D pré ou/et post opératoire de la zone d'intervention. Le fort rendement en photons de scintillation permet de détecter sélectivement les rayons γ pénétrant dans le cristal via le collimateur du dispositif.

Le domaine d'application médicale de l'invention concerne également toute technique pré, per ou post opératoire, nécessitant la recherche de sites ayant été marqués de manière radioactive par un marqueur dopé au moyen d'un radio-isotope émetteur de simple photon d'énergie définie (ou de plusieurs émetteurs gamma d'énergies différentes susceptibles d'être séparés eu égard à la sensibilité et la résolution en énergie du détecteur). Aussi ce dispositif trouve-t-il son application :

- immédiatement avant l'intervention chirurgicale pour remplacer la lymphoscintigraphie traditionnelle effectuée la veille de l'intervention pour détecter et localiser les ganglions sentinelles.
- après l'intervention chirurgicale pour vérifier la qualité de l'exérèse et de s'assurer qu'aucun ganglion sentinelle n'a été laissé en place. Ce contrôle qualité peut se faire pendant la durée nécessaire à l'examen extemporané des ganglions extraits dans un service d'anatomopathologie. Ce dispositif médical s'avère particulièrement pertinent pendant la phase obligatoire d'apprentissage du chirurgien.

Dans des modes de réalisation préférés de l'invention, on peut éventuellement avoir recours en outre à l'une et/ou à l'autre des dispositions suivantes :

- les moyens de traitement électronique sont disposés dans un réceptacle métallique pour assurer une isolation desdits moyens de traitement électronique,
- les moyens de traitement électronique comprennent en outre un ADC adapté pour numériser la charge électrique mesurée, et un processeur comprenant un lien standard de communication adapté pour transmettre les données numérisées par l'ADC vers un micro-ordinateur d'acquisition,
- le cristal scintillant présente une épaisseur inférieure à 4mm,
- les moyens de transformation comprennent au moins un photomultiplicateur multivoies à photocathode bialkali, ledit photomultiplicateur présentant une face de détection placée en regard du cristal scintillant,
- les moyens de transformation comprennent une pluralité de photomultiplicateurs à photocathode bialkali multivoies, chacun desdits photomultiplicateurs présentant une face de détection placée en regard du cristal scintillant,
- des moyens de traitement électronique des signaux électriques produits par les moyens de transformation multivoies, placés à proximité immédiates desdits moyens de transformation multivoies,
- chaque photomultiplicateur multivoies comprend des moyens de traitement électronique propres,
- les moyens de traitement électronique comprennent au moins un multiplexeur analogique pour lire séquentiellement l'ensemble des voies
- le collimateur comprenant sur sa face avant une pluralité de trous traversants aptes à canaliser le rayonnement radioactif,
- une surface réfléchissante est interposée entre le collimateur et le cristal,
- le cristal est en Gadolenium silicate dopé au Cérium (GSO).

L'invention concerne également un procédé de mise en oeuvre du dispositif selon l'invention pour la localisation de tissus marqués par au moins un marqueur radioactif, en particulier pour la localisation de ganglions sentinelles, selon la revendication 12 et comprenant les étapes suivantes :

- mise en place du collimateur à proximité immédiate d'un tissu susceptible d'être marqué,
- détermination de la présence du tissu marqué à partir des signaux électriques fonction notamment des voies ayant été touchées par les photons de scintillation.

Le procédé peut également comprendre l'un des modes de réalisation suivants :

- une image 2D permettant la visualisation des tissus marqués est formée,
- la distance séparant le collimateur des tissus marqués est calculée à partir de la largeur totale à mi hauteur des projections de l'image 2D sur deux axes perpendiculaires de ladite image 2D,
- la distance est déterminée selon la relation $L_{TMH} = 0.3d + 0.5$, avec $L_{TMH}$ la largeur totale à mi hauteur selon l'un des deux axes perpendiculaires, et d est distance entre les tissus marqués et le collimateur.

D'autres caractéristiques et avantages de l'invention apparaîtront encore à la lecture de la description qui va suivre. Celle-ci est purement illustrative et doit être lue en regard des dessins annexés sur lesquels :

- La figure 1 est un schéma de principe d'un dispositif selon l'invention,
- La figure 2 est une vue en perspective et éclatée du dispositif selon l'invention,
- La figure 3 est un schéma de principe du dispositif électronique permettant la discrimination et l'intégration du signal fourni par le photomultiplicateur,
- La figure 4 est une courbe 3D correspondant à la détection des $\gamma$ de 122 keV d'une source de $^{57}$Co émettant isotropiquement ($4\pi$) au moyen du dispositif selon l'invention,
- La figure 5 est une image scintigraphique de deux fantômes au $^{57}$Co de ganglions sentinelles distants de 20 mm et émettant dans $4\pi$ obtenue au moyen du dispositif suivant l'invention.
- La figure 6a est une courbe correspondant à la distribution sur l'axe x de la projection (en nombre de photons de scintillation par unité de temps) de l'image d'un ganglion sentinelle distant de la surface de la peau du patient de 4,3mm,
- La figure 6b est une courbe correspondant à la distribution sur l'axe x de la projection (en nombre de photons de scintillation par unité de temps) de l'image d'un ganglion sentinelle distant de la surface de la peau du patient de 44,3mm,
- La figure 7a présente la variation de la largeur totale à mi-hauteur de la projection dans la direction x de l'image d'un fantôme de ganglion sentinelle d'activité variant entre 10 et 32 kBq, et,
- La figure 7b présente la variation de la largeur totale à mi-hauteur de la projection dans la direction y de l'image d'un fantôme de ganglion sentinelle d'activité variant entre 10 et 32 kBq.

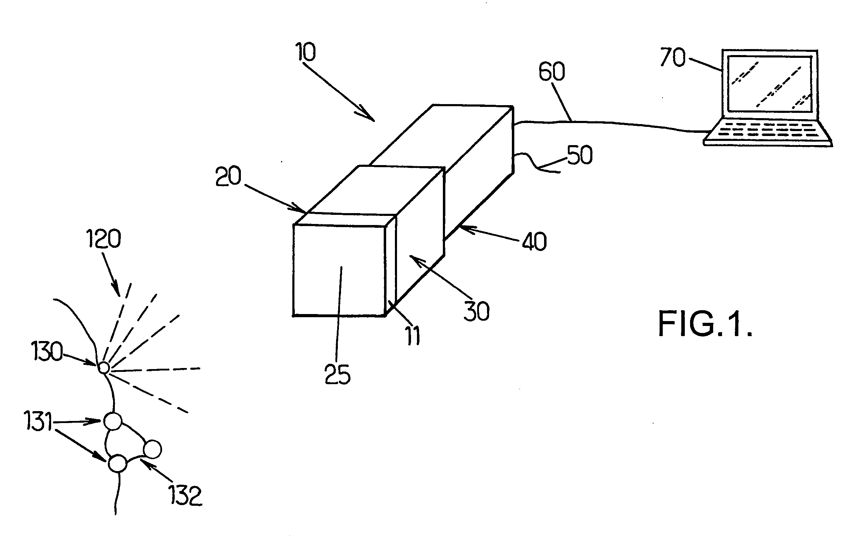

Le principe de réalisation du dispositif selon l'invention est présenté à la figure 1. Il comprend une gamma caméra 10 de forme allongée destinée à la détection des rayons $\gamma$ émis depuis les tissus marqués par un marqueur radioactif. Le marqueur radioactif injecté au patient présente une durée de vie courte, de l'ordre de quelques heures. Il peut être par exemple un radiocolloïde du type sulfure de rhénium contenant le radio nucléide technétium $^{99m}$Tc, émetteur de rayonnement $\gamma$. L'énergie du $\gamma$ émis dans ce cas est de 140,5 keV. Il peut contenir également d'autre source émettrice de rayonnement X ou $\gamma$ comme le Thallium $^{201}$Tl ou l'Iode $^{123}$I. Le radiocolloïde se concentre dans un ou plusieurs ganglions sentinelles 130 du système lymphatique 132 comprenant également d'autres ganglions 131. La gamma caméra 10 comprend un collimateur 11 destiné à sélectionner les rayons $\gamma$ 120 issus du radio isotope dans des directions bien définies. Le collimateur 11 peut par exemple être un collimateur parallèle. La face avant 25 du collimateur 11 constitue la face de détection de la gamma caméra, ou dispositif de détection.

La gamma caméra comprend par ailleurs un cristal scintillant inorganique 20 pour la conversion en photons lumineux de scintillation des rayons $\gamma$ émis 120 par les tissus marqués par un marqueur radioactif. Un blindage (non représenté), en alliage de tungstène par exemple, et entourant le cristal 20, permet de le protéger du bruit radioactif ambiant et assure une bonne détection directionnelle.

Le dispositif comprend par ailleurs des moyens de transformations des photons de scintillation en un signal électrique de détection exploitable, en l'occurrence un photomultiplicateur 30, par effet photoélectrique sur la photocathode. Un dispositif électronique 40 permet la discrimination et l'intégration du signal fourni par le photomultiplicateur 30 au moyen de circuits intégrés dédiés (non représentés). Il permet par ailleurs la sélection du signal intégré correspondant à l'énergie du rayon $\gamma$ du traceur qui a été détecté dans le cristal 20 et la transmission des données ainsi recueillies vers une unité de traitement 70 via un lien électronique 60, comme un lien USB par exemple. Une alimentation 50 est prévue pour alimenter le dispositif selon l'invention.

L'unité de traitement 70 permet de calculer la position en temps réel du rayon $\gamma$ ainsi sélectionné dans le plan de la caméra pour former ensuite une image permettant de dénombrer et de localiser le ou les ganglions sentinelles sources du radio isotope. Un écran de visualisation permet d'afficher en ligne une telle image.

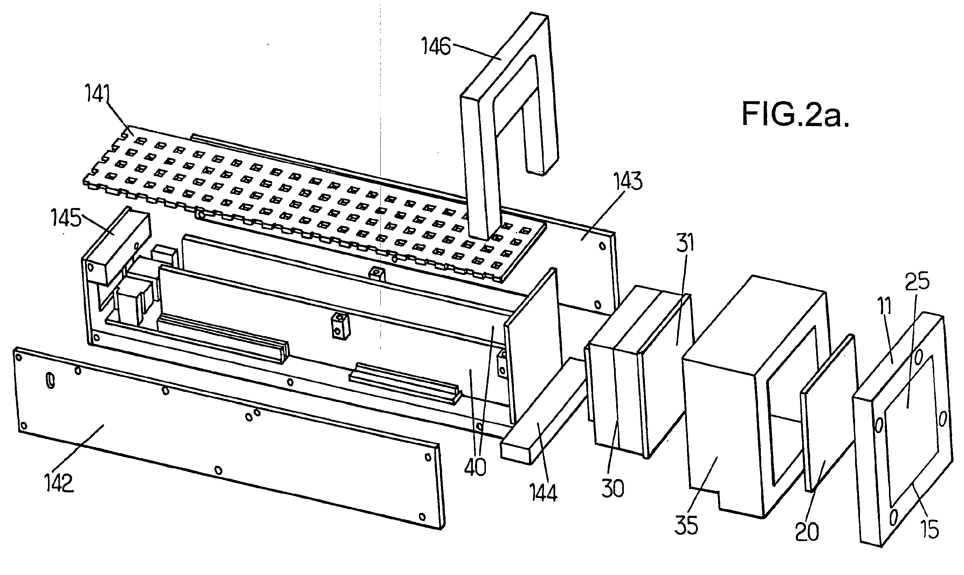

La figure 2 présente une vue éclatée et en perspective du dispositif de détection selon l'invention. Le collimateur 11 correspond à la face de détection de la gamma caméra, et comprend sur sa face avant 25 une pluralité de trous traversants ou orifices 15 aptes à canaliser le rayonnement $\gamma$ vers le cristal 20. Le rôle du collimateur est de canaliser et sélectionner le rayonnement $\gamma$ émis par les radio isotopes et arrivant quasi normalement à la face de détection 25 du dispositif de détection.

Le cristal 20 est plan et est constitué d'un scintillateur inorganique de densité élevée, de numéro atomique élevé et ayant un temps de décroissance rapide. Il présente également un rendement en photons de scintillation élevé afin d'assurer une résolution en énergie suffisante de la caméra. De nombreux cristaux (voir Table 1 ci-dessous) répondent à ces exigences qu'ils soient hygroscopiques (LaCl$_3$ :Ce, LaBr$_3$ :Ce) ou non (GSO :Ce, YAP :Ce, LYSO :Ce).

|  |  | GSO Gd$_2$ SiO$_5$ : Ce Gadolenium silicate dopé au Cérium | YAP YAlO$_3$ : Ce Yttrium Aluminium Perovskite dopé au Cérium | LaCl$_3$: Ce Lanthanum Chloride dopé au Cérium | LaBr3 : Ce Lanthanu m Bromide dopé au Cérium | LYSO Lutetium Yttrium, orthosilicate dopé au Cérium Lu$_{1,8}$ Y$_{0,2}$ SiO$_5$ |
|---|---|---|---|---|---|---|
| à 140 keV | Densité (g.cm$^{-3}$) | 6,71 | 5,37 | 3,79 | 5,29 | 7,1 |
|  | $\mu/\rho$ (cm$^2$.g$^{-1}$) | 0,854 | 0,319 | 0,704 | 0,652 | 1,442 |
|  | 1/$\mu$ (cm) coefficient d'atténuation linéaire | 0,17 | 0,58 | 0,37 | 0,29 | 0,10 |
|  | Z$_{eff}$ | 58 | 36 | 46 | 47 | 65 |
|  | Indice de réfraction | 1,85 | 1,95 | 1,90 | 1,88 | 1,81 |
|  | Temps de décroissance (ns) | 56 | 25 | 28 | 35 | 48 |
|  | Longueur d'onde d'émission (nm) | 430 | 370 | 350 | 358 | 430 |
|  | Photons.MeV$^{-1}$ | 9,0 10$^3$ | 1,8 10$^4$ | 4,9 10$^4$ | 6,3 10$^4$ | 3,2 10$^4$ |

## Table 1 : caractéristiques principales de différents cristaux scintillants

L'épaisseur du cristal peut être choisie pour que la probabilité de détection $\varepsilon_d$ d'un rayon $\gamma$ (en l'occurrence pour le technétium $^{99m}$Tc entrant par un orifice 15 du collimateur 11) soit comprise entre 70 et 90%. Sachant que la longueur d'interaction d'un photon de 140 keV est $\lambda$int $\approx$1,7. mm dans un cristal GSO, un cristal plan de 2mm permet de convertir 70% des photons de désintégration ($\varepsilon_d$ = 0,7). Même si le rendement en photons lumineux reste inférieur à celui du cristal YAP, il permet de proposer une caméra plus compacte (2mm d'épaisseur avec le cristal GSO contre 7mm avec le cristal YAP pour une même probabilité $\varepsilon_d$). Une épaisseur réduite permet également de réduire l'étalement des photons de scintillation arrivant sur la fenêtre d'entrée du photomultiplicateur. Un signal plus confiné permet une localisation accrue de la position des ganglions sentinelles. En effet pour un dénombrement et une localisation efficaces des ganglions sentinelles, il est nécessaire que la caméra selon l'invention offre une détection élevée et une résolution en position (pouvoir séparateur entre les ganglions sentinelles proches) élevées. Une excellente résolution en énergie n'est pas un paramètre pertinent pour la fiabilité de la détection. La gamma caméra trouve donc particulièrement son application pour les applications SPECT (de l'anglais Single Photon Emission Computed Tomography). Elle peut également être utilisée avec un cristal tel que le LYSO d'épaisseur adaptée dans les applications PET (de l'anglais Positron Emission Tomography), pour des photons de 511 keV.

Le cristal 20 est interposé entre le collimateur 11 et le photomultiplicateur 30. Le collimateur 11 est attaché à un blindage extérieur 35 qui présente une ouverture traversante pour recevoir le photomultiplicateur 30. Le cristal 20 est maintenu entre le collimateur 11 et le blindage, et le photomultiplicateur est inséré dans l'ouverture traversante jusqu'à ce que sa face de détection 31 entre en contact avec le cristal.

Une surface réfléchissante, comme par exemple une feuille de mylar aluminisé, peut également être interposée entre le collimateur 11 et le cristal 20. La face d'entrée du cristal 20 peut également avoir reçu un traitement de surface réfléchissant.

Le photomultiplicateur 30 est un photomultiplicateur multivoies plan. Il présente donc une face de détection 31 photosensible à la position des photons arrivant sur cette face. Le photomultiplicateur est du type multianode, ce qui permet d'obtenir grâce au dispositif électronique 40 et à l'unité de traitement 70 une image des ganglions sentinelles détectés. Les photoélectrons produits sur la photocathode sont dirigés électrostatiquement vers des régions d'amplification homothétiques de leur point de conversion sur la face 31. Le photomultiplicateur présente sur sa fenêtre de détection 31 un verre plan destiné à venir en contact avec le cristal par le biais par exemple d'une graisse optique, d'un silicone ou d'une colle optique, lorsque le photomultiplicateur est inséré dans le blindage 35. En fonction de la taille du cristal, et de la surface de détection de la gamma caméra, plusieurs photomultiplicateurs multivoies, comme expliqué plus loin, peuvent être nécessaires.

Le dispositif électronique 40, détaillé ci après, est lui-même relié au photomultiplicateur 30. Il est disposé dans un réceptacle formée de deux parois latérales 142 et 143, d'un support 144 et d'une paroi supérieure 141, tous préférablement métalliques pour assurer une isolation du dispositif électronique 40. Une bague de maintien 146, ainsi qu'un couvercle 145 viennent fermer le réceptacle. Le couvercle 145 présente une ouverture pour le lien électronique 60. Le dispositif électronique constitue donc des moyens de traitement électronique 40 des signaux électriques produits par les moyens de transformation multivoies 30, et sont placés à proximité immédiate des moyens de transformation multivoies.

Une étude ex vivo de ganglions sentinelles au moyen d'un compteur γ au moment de leur excision a montré que leur activité moyenne est de l'ordre de 7kBq [0,04 - 56,0]. Il a également été montré que la radioactivité peut être considérée comme uniformément répartie à l'intérieur du ganglion sentinelle conformément à la population cellulaire ganglionnaire. Les dimensions d'un ganglion sentinelle moyen sont infra centimétriques et l'émission des rayons γ de désintégration est isotrope.

Ces constations permettent de dimensionner la surface de détection du dispositif selon l'invention. Compte tenu de l'étendue du creux axillaire, une fenêtre d'environ 100 x 100 mm$^2$ est appropriée pour avoir une bonne détection des ganglions sentinelles dans cette région anatomique. Le photomultiplicateur multivoies dépend de la taille de la surface de détection 25 de la gamma caméra. On peut notamment associer plusieurs photomultiplicateurs multivoies en parallèle pour couvrir la fenêtre de 100 x 100 mm$^2$ proposée.

En fonction de la taille choisie de la fenêtre, et donc du cristal plan en regard du collimateur, on peut assembler ce dernier à partir de cristaux plus petits, ou lorsque cela est réalisable, faire croître un tel cristal jusqu'à la taille souhaitée. Par exemple, le cristal de 100 x 100 mm$^2$ peut être reconstitué à partir d'un assemblage de 16 cristaux de 25 x 25 mm$^2$ ou de 4 cristaux de 50 x 50 mm$^2$ placés sur la fenêtre du photomultiplicateur.

La résolution spatiale du dispositif selon l'invention est égale à $R_s = \sqrt{R_i{}^2 + R_c{}^2}$ où $R_i$ et $R_c$ représentent respectivement la résolution spatiale intrinsèque du dispositif et la résolution spatiale du collimateur.

Pour le collimateur, $Rc = \dfrac{(d+H) \times e}{H - 2/\mu}$, avec d la distance entre la source radioactive et la face d'entrée du collimateur, e le diamètre des orifices, H l'épaisseur du collimateur, et $\mu$ le coefficient d'atténuation linéaire du matériau constituant le collimateur. L'efficacité géométrique de détection est quant à elle fonction du diamètre e des orifices du collimateur, de H, de la taille des septas s (quantité de matière séparant deux orifices successifs) et s'exprime sous la forme :

$$\varepsilon_g = \frac{\sqrt{3}}{8\pi}\left[\frac{e^2}{(H - 2/\mu)(e+s)}\right]$$

Les 3 paramètres e, H et s sont déterminants pour optimiser l'efficacité de détection tout en conservant une résolution spatiale suffisante pour distinguer deux ganglions sentinelles proches. Par exemple, en supposant que la distance moyenne d'un ganglion sentinelle à l'entrée du système est d+H = 50mm, e = 1,6mm, et s = 0,2mm, la résolution spatiale $R_s$ du système est de l'ordre de 10mm. Dans ces mêmes conditions, l'efficacité $\varepsilon_g$ est égale à 2.3.10$^{-3}$, conduisant à une sensibilité de la gamma caméra de 1,6 cps.kBq$^{-1}$.

En plus de la localisation des ganglions sentinelles avec une résolution spatiale de 10mm, le système permet de donner une estimation de la profondeur d'un ganglion sentinelle dans le corps humain (distance à la peau). Cette information est d'importance pour le chirurgien, car elle permet de limiter la morbidité du geste opératoire.

La prédiction de la position en profondeur d'un ganglion sentinelle peut être obtenue par la mesure de la largeur totale à mi-hauteur ($L_{TMH}$) des projections de l'image du ganglion sentinelle repérées sur les axes x et y (plan xy de détection). En effet, cette largeur Ltmh dépend de la distance du ganglion sentinelle repérée à la phase d'entrée du collimateur. Les études menées en laboratoire avec des fantômes de ganglions sentinelles montrent que la largeur des distributions dans les directions x et y des projections varient linéairement en fonction de la distance au collimateur du système selon la relation :

$$L_{TMH} \text{ (en mm)} \approx 0.3 \, d \text{ (mm)} + 5 \qquad\qquad (1)$$

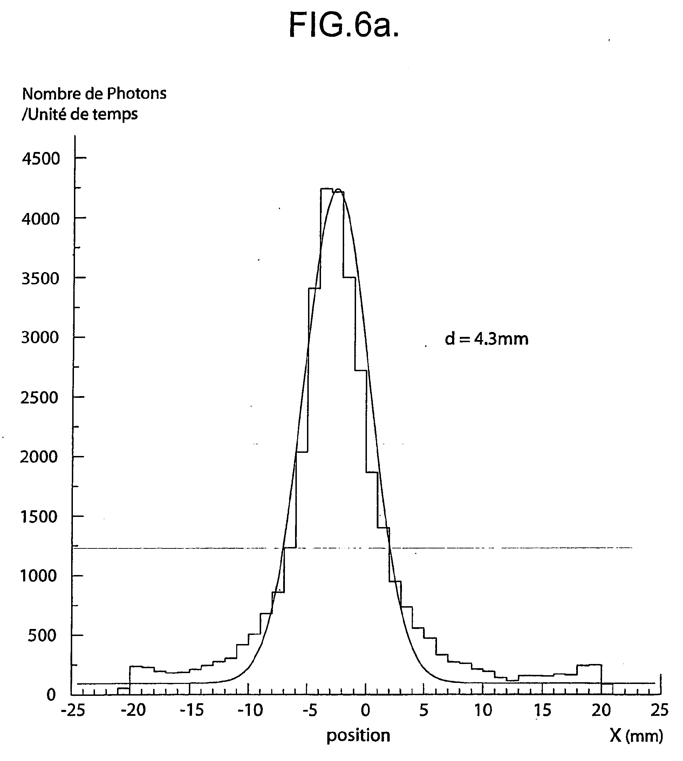

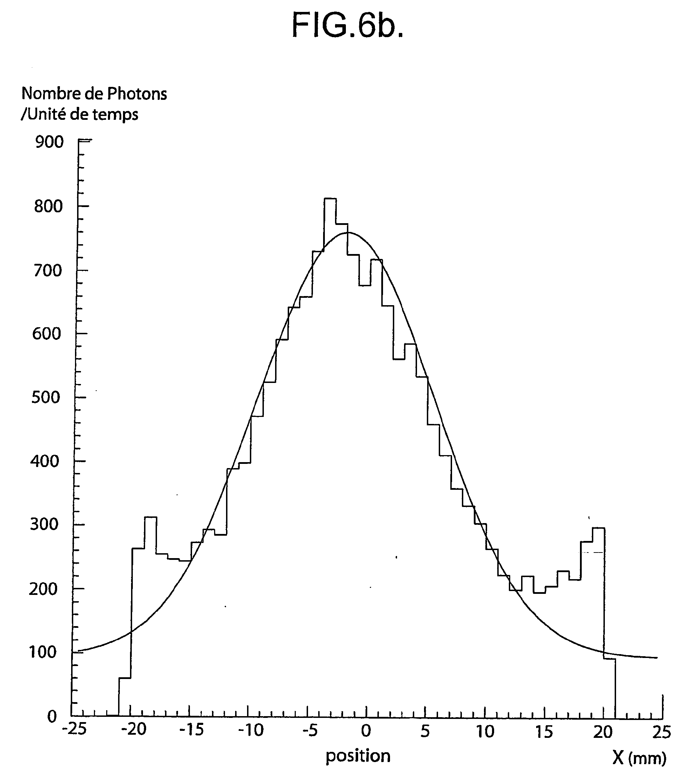

Les figures 6a et 6b montrent les histogrammes de projections (par exemple dans la direction x, dans cet exemple les projections correspondent au nombre de photons de scintillation compté par unité de temps) de l'image d'un fantôme de ganglions sentinelles située respectivement à 4,3 mm et 44,3 mm du collimateur. La valeur de la largeur totale à mi-hauteur $L_{TMH}$ peut être obtenue après ajustement d'une gaussienne sur cette projection comme on peut le constater aux figures 6a et 6b.

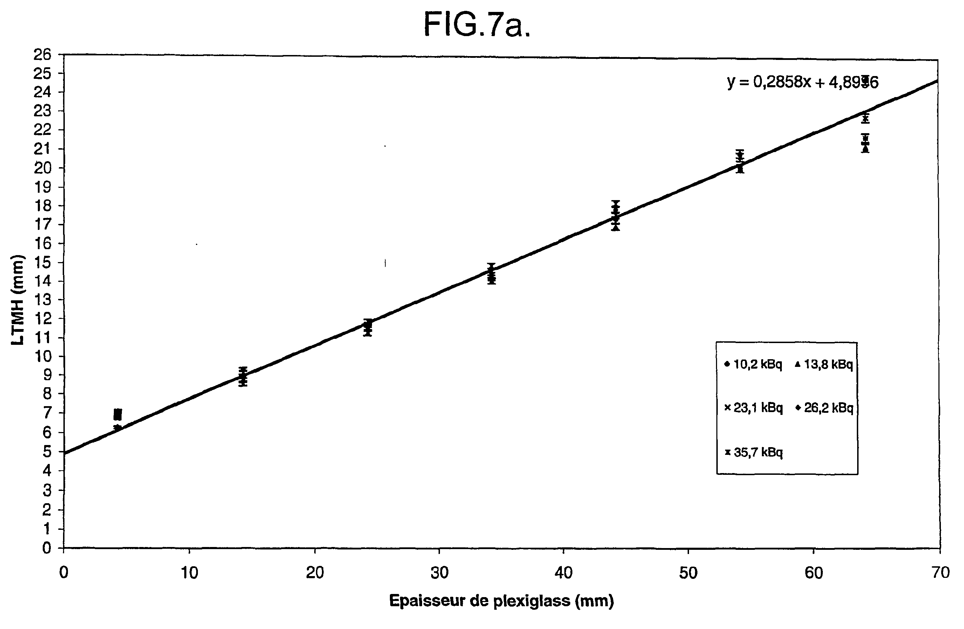

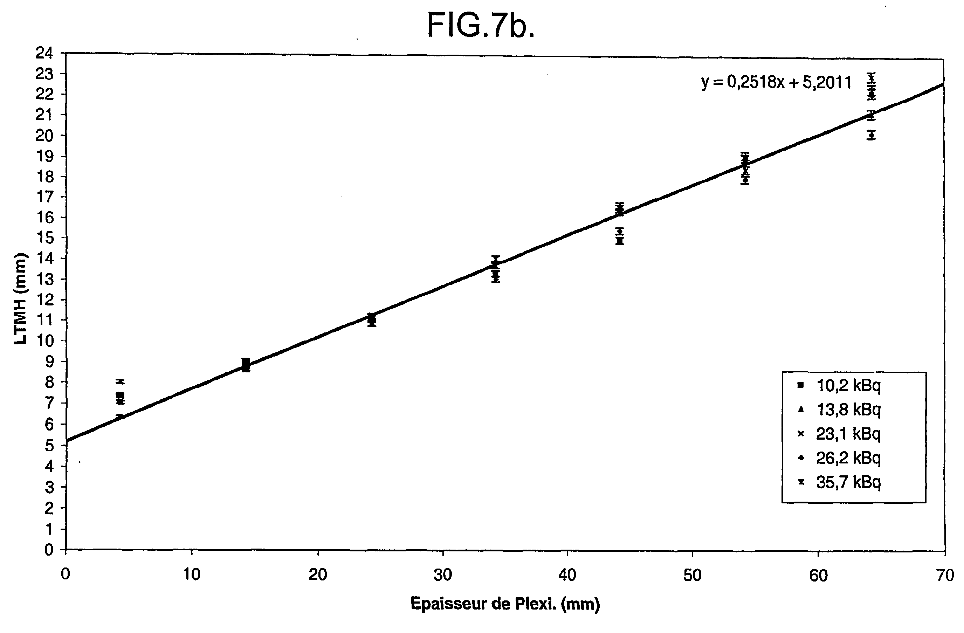

Les figures 7a et 7b présentent les variations de $L_{TMH}$ selon x et y respectivement déterminées à partir des projections de fantômes de ganglions sentinelles présentant des activités variant entre 10 et 32 kBq. La contribution de la diffusion du rayonnement gamma par les tissus a été simulée en utilisant des épaisseurs variables de plexiglas. La relation (1) a pu être déterminée à partir des deux droites de corrélation visibles aux figures 7a et 7b respectivement.

Le système selon l'invention permet donc à partir de la relation (1) de déterminer la position en profondeur d'un ganglion sentinelle.

Les tissus marqués du ganglion sentinelle émettent un rayonnement radioactif isotrope ($4\pi$). Le collimateur 11 du dispositif selon l'invention, lorsqu'il est placé en regard du ou des ganglions sentinelles, permet de sélectionner au moins une direction définie, en l'occurrence une direction normal au cristal grâce aux orifices parallèles. En effet, les rayonnements qui s'éloignent sensiblement de cette direction viennent frapper le collimateur et sont absorbés. Les rayonnements canalisés par les orifices viennent frapper le cristal scintillant 20, qui transforment ces rayonnements quand ils ont interagi en des photons de scintillation. Ces photons lumineux se propagent dans le cristal et viennent frapper la fenêtre d'entrée du photomultiplicateur sur une zone dont l'étendue dépend notamment de l'épaisseur du cristal. Grâce aux choix d'un cristal comme le GSO, cette épaisseur est limitée, et la diffusion des photons de scintillation au regard de chaque orifice reste limitée.

Le choix d'un photomultiplicateur multivoie permet une discrétisation géométrique de la face de détection du photomultiplicateur en regard du cristal. Le photomultiplicateur correspond à des moyens de transformation multivoies des photons de scintillation en signaux électriques de détection, ces signaux électriques étant fonction notamment des voies ayant été touchées par les photons de scintillation. Ainsi les photons de scintillation issus du rayonnement canalisé dans un orifice peuvent être localisés sur la face de détection. Le dispositif électronique 40, associé à l'unité de traitement 70 permet de transformer les signaux électriques de détection issu du photomultiplicateur 30 en une image pixélisée indiquant la position du ou des orifices qui ont canalisé le rayonnement, et donc la position du ou des ganglions sentinelles en regard de la gamma caméra. Dans la pratique, des photomultiplicateurs de type multianode (à 64 voies) peuvent être utilisés pour obtenir une image pixélisée du ganglion sentinelle.

En fonction de la taille du cristal, un ou une pluralité de photomultiplicateurs multivoies peuvent être nécessaires, ils sont alors juxtaposés les uns à côté des autres pour que l'ensemble de la surface du cristal soit en regard de la face de détection d'un photomultiplicateur.

Le nombre important de signaux à traiter et la nécessité de réaliser une gamma caméra à faible encombrement obligent à recourir à une électronique intégrée pour le traitement analogique des signaux. Il sera préférable de placer l'ensemble de l'électronique au plus proche du photodétecteur et de rendre cette électronique communicante avec un ordinateur distant via un lien standard de communication (USB par exemple). L'ensemble doit rester suffisamment compact pour conserver un encombrement final acceptable de la gamma caméra. Celle-ci doit en effet pouvoir être placée par exemple en regard du creux axillaire lors d'interventions chirurgicales sur le sein. Ce dispositif doit comprendre, en dehors de l'électronique intégrée de traitement des signaux, l'alimentation haute tension du photodétecteur, un moyen de conversion analogique numérique des mesures de charges fournies par les voies illuminées du photodétecteur et des éléments de contrôle des paramètres de fonctionnement de l'électronique (seuils de déclenchement, niveau de haute tension, etc). Un dispositif numérique (processeur par exemple) devra également être implémenté pour assurer un contrôle et un formatage des données avant leur transmission à l'ordinateur distant. La partie intégrée de cette électronique doit traiter les signaux du photodétecteur qui peuvent être, dans le cas de l'utilisation d'un cristal à temps de décroissance rapide, inférieurs en durée à 200 ns. Elle comprendra pour chaque voie un dispositif de déclenchement permettant d'indiquer par un signal le moment où une voie du photodétecteur est illuminée. Une mesure des charges fournies par le photodétecteur doit suivre chacun de ces déclenchements. Cette mesure sera réalisée indépendamment pour chacune des voies au moyen d'un dispositif d'intégration permettant de mesurer l'ensemble de la charge dont l'étalement en temps dépend du type de cristal utilisé (au minimum 200 ns). Les données ainsi obtenues seront transférées à l'ordinateur distant qui, après un traitement logiciel, fournira en temps réel l'image scintigraphique.

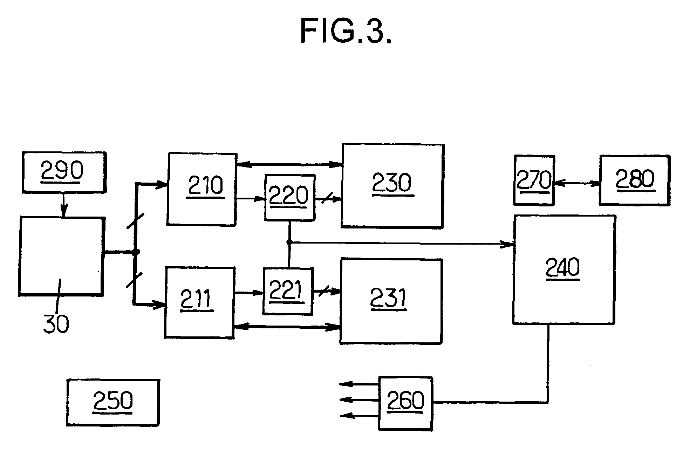

La figure 3 est un schéma de principe d'un tel dispositif électronique permettant la discrimination et l'intégration du signal fourni par le (ou les) photomultiplicateur(s).

Le dispositif vient s'enficher directement derrière le ou les photomultiplicateur(s) et comprend l'ensemble des éléments nécessaires au traitement des signaux jusqu'à l'obtention des données informatiques. Le faible encombrement de cette électronique permet d'avoir une gamma caméra très maniable et bien adaptée aux zones à observer, en particulier le creux axillaire. Le photomultiplicateur, ou chaque photomultiplicateur lorsque plusieurs sont nécessaires, possède en réalité une électronique propre indépendante. Le boîtier représenté à la figure 2 permet de recevoir l'électronique associée à un photomultiplicateur.

La description qui suit reprend l'exemple d'un photomultiplicateur 64 voies comme mentionné précédemment. Deux chaînes de traitement 32 voies permettent de traiter les 64 voies du photomultiplicateur. Ces chaînes comprennent notamment un circuit intégré (respectivement 210 et 211) conçu pour le traitement de ce type de photomultiplicateur. Chaque circuit intégré contient :

- un premier amplificateur à gain ajustable qui permet de compenser les dispersions de gains entre les différentes voix d'un même photomultiplicateur,
- une étape d'intégration du signal à l'issue de laquelle une mesure précise de la charge est possible,
- un discriminateur fournissant un signal logique de déclenchement à chaque fois que la cellule correspondante est illuminée par des photons de scintillation.

Un multiplexeur analogique en sortie (non représenté) de ce circuit électronique assure une lecture séquentielle des 32 voies. Un seul ADC (Analog to Digital Converter, respectivement 220 et 221) est suffisant pour numériser la charge mesurée recueillie sur chacune des 32 voies. L'ensemble de la logique nécessaire au fonctionnement de chaque circuit intégré est réalisé par un FGPA (Field Programmable Gate Array, respectivement 230 et 231). Les données produites par les ADC 220 et 221 sont lues par un processeur 240 qui, via un lien standard USB (Universal Serial Bus 270) les transmet à un micro-ordinateur 280 d'acquisition. Cette communication avec le micro-ordinateur 280 est également utilisée pour contrôler l'ensemble des paramètres de fonctionnement du photomultiplicateur 30 via une interface utilisateur. Le photomultiplicateur 30 est alimenté par une alimentation miniature haute tension 290 pouvant délivrer jusqu'à 1000 V pour un courant inférieur à 0.5 mA. Un DAC (Digital to Analog Converter 260) permet de fixer la valeur de cette haute tension, ainsi que les valeurs du seuil de discrimination de l'énergie des photons de scintillation. Pour alimenter cette électronique du photomultiplicateur, une alimentation 250 du type 9 V est suffisante, toutes les tensions nécessaires au fonctionnement des différents composants étant créées à partir de celle-ci.

La position de scintillation du rayon gamma incident peut être reconstruite à partir d'un algorithme de groupement (de l'anglais clustering algorithm), en prenant en compte la charge collectée sur toutes les voies des photomultiplicateurs.

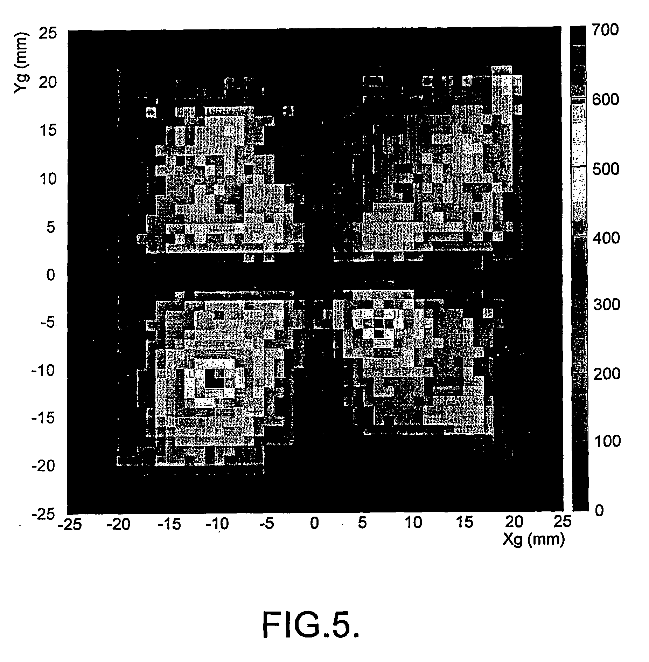

Les ports USB nécessaires pour l'ensemble des photomultiplicateurs peuvent être regroupés entre eux au moyen d'un concentrateur afin de limiter à un seul câble la liaison entre la gamma caméra selon l'invention et le micro-ordinateur d'acquisition. Un logiciel est prévu sur ce micro-ordinateur pour le contrôle de l'appareil et l'exploitation des données ainsi que la visualisation en temps réel de l'image scintigraphique, comme dans l'exemple proposé à la figure 5. Une sauvegarde sous forme de fichier informatique de ces données peut être proposée, ce fichier pouvant être joint au dossier médical du patient.

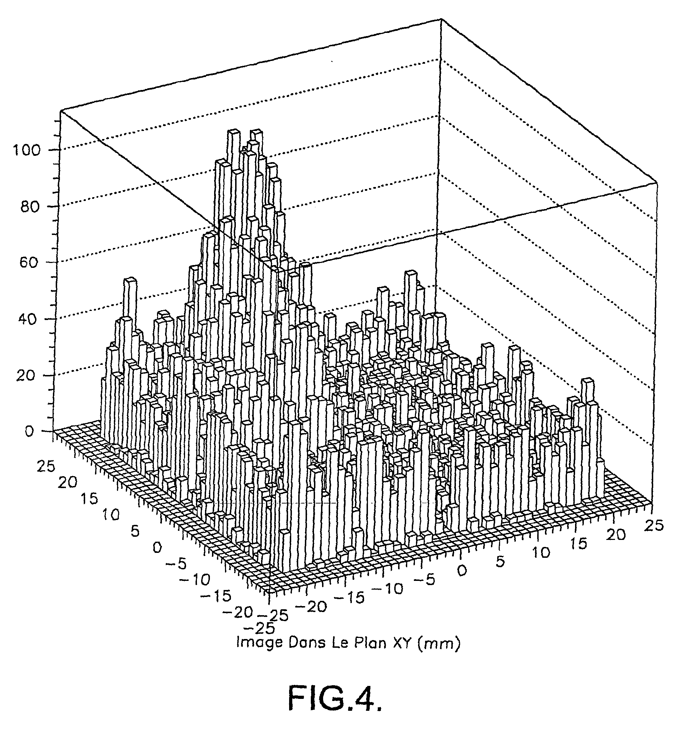

Un exemple de résultat de détection est présenté à la figure 4, sous forme d'un graphe 3D, ou « lego plot », avec pour plan xy le plan de détection, et pour coordonnée z le nombre d'événements/mm$^2$, c'est-à-dire le nombre de $\gamma$ dénombrés par mm$^2$. La source est une source de $^{57}$Co, d'une activité de 3,4 kBq (comparable à l'activité moyenne d'un ganglion sentinelle) et émettant dans $4\pi$ et elle est mesurée avec un dispositif correspondant aux dimensions précisées précédemment. La source est placée à 50mm du collimateur, ici en plomb. Le signal mesuré est de l'ordre de 1,2. $10^{-2}$ mm$^{-2}$ s$^{-1}$ en regard de la source pour un bruit de fond de 3,9.$10^{-3}$ mm$^{-2}$ s$^{-1}$. La résolution en position (définie comme la largeur totale à mi-hauteur du profil de détection) est de l'ordre de 8,5mm en accord avec l'estimation théorique Rs et apparaît comme indépendante du point d'impact du rayon $\gamma$ sur le cristal. La sensibilité du détecteur est de l'ordre de 1,1 cps.kBq$^{-1}$ en accord avec son estimation théorique. La figure 5 représente l'image scintigraphique de deux fantômes de ganglions sentinelles d'activité 3,6 kBq et 6,8 kBq distants de 20 mm obtenue avec le dispositif présenté sur la figure 2.

**Revendications**

1. Dispositif pour la localisation de tissus marqués (130), en particulier pour la localisation de ganglions sentinelles, comprenant :

   - un collimateur (11) apte à sélectionner un rayonnement radioactif émis par des tissus marqués (130) par au moins un marqueur radioactif dans au moins une direction définie, ledit collimateur étant attaché à un blindage extérieur ;
   - au moins un cristal scintillant (20) pour transformer en photons de scintillation ledit rayonnement radioactif (120), ledit cristal scintillant étant entouré par le blindage pour le protéger du bruit radioactif ambiant ; et le cristal scintillant étant un cristal inorganique préférablement non hygroscopique, présentant un rendement en photons de scintillation supérieur à 9000 photons/MeV,
   - des moyens de transformation multivoies (30) desdits photons de scintillation en signaux électriques de détection, lesdits signaux électriques étant fonction notamment des voies ayant été touchées par lesdits photons de scintillation, lesdits moyens de transformation multivoies étant insérés dans le blindage ; **caracterisé par**
   - des moyens de traitement électronique (40) des signaux électriques produits par les moyens de transformation multivoies, lesdits moyens de traitement étant placés à proximité immédiate et directement derrière desdits moyens de transformation multivoies pour que le dispositif ait un faible encombrement et soit maniable, et comprenant au moins un circuit intégré comprenant un étage d'intégration desdits signaux électriques adapté pour fournir une mesure de la charge électrique desdits signaux électriques en une durée inférieure à 200 ns.

2. Dispositif selon la revendication précédente, dans lequel les moyens de traitement électronique (40) sont disposés dans un réceptacle métallique pour assurer une isolation desdits moyens de traitement électronique (40).

3. Dispositif selon l'une des revendications précédentes, dans lequel les moyens de traitement électronique (40) comprennent en outre :

   - un ADC (220, 221) adapté pour numériser la charge électrique mesurée, et
   - un processeur (240) comprenant un lien standard de communication adapté pour transmettre les données numérisées par l'ADC (220, 221) vers un micro-ordinateur (280) d'acquisition.

4. Dispositif selon l'une des revendications précédentes, dans lequel ledit cristal scintillant présente une épaisseur inférieure à 4mm.

5. Dispositif selon l'une des revendications précédentes, dans lequel les moyens de transformation (30) comprennent au moins un photomultiplicateur multivoies à photocathode bialkali, ledit photomultiplicateur présentant une face de détection (31) placée en regard du cristal scintillant.

6. Dispositif selon la revendication précédente, dans lequel les moyens de transformation (30) comprennent une pluralité de photomultiplicateurs à photocathode bialkali multivoies, chacun desdits photomultiplicateurs présentant une face de détection (31) placée en regard du cristal scintillant,

7. Dispositif selon les revendications 5 ou 6, dans lequel chaque photomultiplicateur multivoies comprend des moyens de traitement électronique propres (40).

8. Dispositif selon l'une des revendications 1 ou 7, dans lequel les moyens de traitement électronique comprennent au moins un multiplexeur analogique pour lire séquentiellement l'ensemble des voies.

9. Dispositif selon l'une des revendications précédentes, dans lequel le collimateur comprend sur sa face avant (25) une pluralité de trous traversants (15) aptes à canaliser le rayonnement radioactif.

10. Dispositif selon l'une des revendications précédentes, dans lequel une surface réfléchissante est interposée entre le collimateur et le cristal.

11. Dispositif selon l'une des revendications précédentes, dans lequel le cristal est en Gadolenium Silicate dopé au Cérium (GSO).

12. Procédé de localisation de tissus marqués (130) par au moins un marqueur radioactif, en particulier pour la localisation de ganglions sentinelles, à partir du dispositif selon l'une des revendications 1 à 11, et comprenant les étapes suivantes :

- mise en place du collimateur à proximité immédiate d'un tissu susceptible d'être marqué,
- détermination de la présence du tissu marqué à partir des signaux électriques fonction notamment des voies ayant été touchées par les photons de scintillation.

**13.** Procédé selon la revendication précédente, comprenant une étape supplémentaire de formation d'une image 2D permettant la visualisation des tissus marqués.

**14.** Procédé selon la revendication précédente, comprenant une étape supplémentaire de calcul de la distance séparant le collimateur des tissus marqués à partir de la largeur totale à mi hauteur des projections de l'image 2D sur deux axes perpendiculaires de ladite image 2D.

**15.** Procédé selon la revendication précédente, dans lequel la distance est déterminée selon la relation :

$$L_{TMH} = 0.3d + 0.5$$

dans laquelle :

$L_{TMH}$ est la largeur totale à mi hauteur selon l'un des deux axes perpendiculaires,
d est distance entre les tissus marqués et le collimateur.

**Claims**

**1.** A device for locating marked tissues (130), in particular for locating sentinel nodes, comprising:

- a collimator (11) capable of selecting a radioactive radiation emitted by tissues (130) marked by at least one radioactive marker in at least one defined direction, said collimator being attached to an external shielding;
- at least one scintillation crystal (20) for converting said radioactive radiation (120) into scintillation photons, said scintillation crystal being surrounded by the shielding to protect it from ambient radioactive noise; and the scintillation crystal being a preferably non-hygroscopic inorganic crystal having a scintillation photons yield of higher than 9000 photons/MeV,
- multi-channel transformation means (30) for transforming said scintillation photons into electrical detection signals, said electrical signals being a function in particular of the channels having been touched by said scintillation photons, said multi-channel transformation means being inserted in the shielding; **characterised by**
- electronic processing means (40) for processing the electrical signals produced by the multi-channel transformation means, said processing means being placed in the immediate proximity of and directly behind said multi-channel transformation means so that the device is compact and manageable, and comprising at least one integrated circuit comprising a stage for integration of said electrical signals that is adapted to provide a measurement of the electrical charge of said electrical signals in a period of less than 200 ns.

**2.** A device according to the preceding claim wherein the electronic processing means (40) are disposed in a metal receptacle for ensuring isolation of said electronic processing means (40).

**3.** A device according to one of the preceding claims wherein the electronic processing means (40) further comprise:

- an ADC (220, 221) adapted to digitise the measured electrical charge, and
- a processor (240) comprising a standard communication link adapted to transmit the data digitised by the ADC (220, 221) to an acquisition microcomputer (280).

**4.** A device according to one of the preceding claims wherein said scintillation crystal is of a thickness of less than 4 mm.

**5.** A device according to one of the preceding claims wherein the transformation means (30) comprise at least one multi-channel bialkali photocathode photomultiplier, said photomultiplier having a detection face (31) disposed facing the scintillation crystal.

6. A device according to the preceding claim wherein the transformation means (30) comprise a plurality of multi-channel bialkali photocathode photomultipliers, each of said photomultipliers having a detection face (31) disposed facing the scintillation crystal.

7. A device according to claim 5 or claim 6 wherein each multi-channel photomultiplier comprises its own electronic processing means (40).

8. A device according to one of claims 1 and 7 wherein the electronic processing means comprise at least one analog multiplexer for sequentially reading all of the channels.

9. A device according to one of the preceding claims wherein the collimator comprises on its front face (25) a plurality of through holes (15) adapted to channel the radioactive radiation.

10. A device according to one of the preceding claims wherein a reflecting surface is interposed between the collimator and the crystal.

11. A device according to one of the preceding claims wherein the crystal is of cerium-doped gadolinium silicate (GSO).

12. A process for locating tissues (130) marked by at least one radioactive marker, in particular for locating sentinel nodes, using the device according to one of claims 1 to 11 and comprising the following steps:

- placing the collimator in the immediate proximity of a tissue capable of being marked, and
- determining the presence of the marked tissue from the electrical signals as a function in particular of the channels having been touched by the scintillation photons.

13. A process according to the preceding claim comprising an additional step of forming a 2D image permitting visualisation of the marked tissues.

14. A process according to the preceding claim comprising an additional step of calculating the distance separating the collimator from the marked tissues from the total width at mid height of the projections of the 2D image on to two perpendicular axes of said 2D image.

15. A process according to the preceding claim wherein the distance is determined in accordance with the relationship:

$$L_{TMH} = 0.3d + 0.5$$

in which
$L_{TMH}$ is the total width at mid height along one of the two perpendicular axes, and
d is the distance between the marked tissues and the collimator.

**Patentansprüche**

1. Einrichtung zur Lokalisierung von markierten Geweben (130), insbesondere zur Lokalisierung von Wächter-Lymphknoten, umfassend:

- einen Kollimator (11), der dazu geeignet ist, eine radioaktive Strahlung auszuwählen, welche von Geweben (130) ausgestrahlt wird, die durch mindestens einen radioaktiven Marker markiert sind, in wenigstens einer definierten Richtung, wobei der Kollimator an einer äußeren Abschirmung befestigt ist,
- wenigstens einen Szintillationskristall (20), um die radioaktive Strahlung (120) in Szintillations-Photonen umzuwandeln, wobei der Szintillationskristall von der Abschirmung umgeben ist, um ihn von dem umgebenden radioaktiven Rauschen zu schützen, und wobei der Szintillationskristall ein anorganischer Kristall ist, der bevorzugt nicht hygroskopisch ist, und der eine Ausbeute an Szintillations-Photonen von mehr als 9000 Photonen/MeV aufweist,

- Mehrkanal-Transformationsmittel (30), um die Szintillations-Photonen in elektrische Detektionssignale umzuwandeln, wobei die elektrischen Signale insbesondere von den Kanälen abhängen, die von den Szintillations-Photonen getroffen worden sind, wobei die Mehrkanal-Transformationsmittel in die Abschirmung eingefügt sind, **gekennzeichnet durch**

- Mittel zur elektronischen Verarbeitung (40) der elektrischen Signale, die von den Mehrkanal-Transformationsmittel erzeugt worden sind, wobei die Mittel zur Verarbeitung in unmittelbarer Nähe und direkt hinter den Mehrkanal-Transformationsmitteln angeordnet sind, damit die Einrichtung einen geringen Platzbedarf aufweist und handlich ist, und umfassend wenigstens einen integrierten Schaltkreis, welcher eine Integrationsstufe für die elektrischen Signale umfasst, angepasst, um ein Maß für die elektrische Ladung der elektrischen Signale in einer Zeitdauer von kleiner als 200 ns zu liefern.

2. Einrichtung nach dem vorhergehenden Anspruch, wobei die Mittel zur elektronischen Verarbeitung (40) in einer metallischen Aufnahme angeordnet sind, um eine Isolation der Mittel zur elektronischen Verarbeitung (40) sicherzustellen.

3. Einrichtung nach einem der vorhergehenden Ansprüche, wobei die Mittel zur elektronischen Verarbeitung (40) weiterhin umfassen:

- einen Analog-Digital-Wandler (220, 221), der angepasst ist, um die gemessene elektrische Ladung zu digitalisieren und
- einen Prozessor (240), der eine Standard-Kommunikationsverbindung umfasst, welche angepasst ist, um die durch den Analog-Digital-Wandler (220, 221) digitalisierten Daten zu einem Erfassungs-PC (280) zu übertragen.

4. Einrichtung nach einem der vorhergehenden Ansprüche, wobei der Szintillationskristall eine Dicke von kleiner als 4 mm aufweist.

5. Einrichtung nach einem der vorhergehenden Ansprüche, wobei die Transformationsmittel (30) wenigstens einen Mehrkanal-Photovervielfacher mit einer Bialkali-Photokathode umfassen, wobei der Photovervielfacher eine Detektionsfläche (31) aufweist, die dem Szintillationskristall gegenüber platziert ist.

6. Einrichtung nach dem vorhergehenden Anspruch, wobei die Transformationsmittel (30) eine Mehrzahl von Mehrkanal-Photovervielfachern mit Bialkali-Photokathode aufweisen, wobei jeder der Photovervielfacher eine Detektionsfläche (31) aufweist, welche gegenüber von dem Szintillationskristall platziert ist.

7. Einrichtung nach den Ansprüchen 5 oder 6, wobei jeder Mehrkanal-Photovervielfacher eigene Mittel zur elektronischen Verarbeitung (40) umfasst.

8. Einrichtung nach einem der Ansprüche 1 oder 7, wobei die Mittel zur elektronischen Verarbeitung wenigstens einen Analogmultiplexer umfassen, um sequenziell die Gesamtheit der Kanäle auszulesen.

9. Einrichtung nach einem der vorhergehenden Ansprüche, wobei der Kollimator auf seiner Vorderseite (25) eine Mehrzahl von Durchgangslöchern (15) umfasst, die geeignet sind, die radioaktive Strahlung zu kanalisieren.

10. Einrichtung nach einem der vorhergehenden Ansprüche, wobei eine reflektierende Fläche zwischen dem Kollimator und dem Kristall angeordnet ist.

11. Einrichtung nach einem der vorhergehenden Ansprüche, wobei der Kristall aus Cer-dotiertem Gadoliniumsilikat (GSO) besteht.

12. Verfahren zur Lokalisierung von Geweben (130), die durch wenigstens einen radioaktiven Marker markiert sind, insbesondere für die Lokalisierung von Wächter-Lymphknoten, ausgehend von der Einrichtung gemäß einem der Ansprüche 1 bis 11, und umfassend die folgenden Schritte:

- Positionieren des Kollimators in unmittelbarer Nähe eines Gewebes, welches geeignet ist, markiert zu sein,
- Bestimmung des Vorhandenseins des markierten Gewebes ausgehend von elektrischen Signalen, welche insbesondere von den Kanälen abhängen, die von den Szintillations-Photonen getroffen worden sind.

13. Verfahren nach dem vorhergehenden Anspruch, umfassend einen zusätzlichen Schritt der Bildung eines zweidimensionalen Bildes, welches die Visualisierung der markierten Gewebe ermöglicht.

14. Verfahren nach dem vorhergehenden Anspruch, umfassend einen zusätzlichen Schritt der Berechnung des Abstands, welcher den Kollimator von den markierten Geweben trennt, ausgehend von der Halbwertsbreite der Projektionen des zweidimensionalen Bildes auf zwei senkrechte Achsen des zweidimensionalen Bildes.

15. Verfahren nach dem vorhergehenden Anspruch, wobei der Abstand gemäß der Beziehung bestimmt wird:

$$L_{TMH} = 0,3d + 0,5$$

wobei: $L_{TMH}$ die Halbwertsbreite gemäß einer der zwei senkrechten Achsen ist, und d der Abstand zwischen den markierten Geweben und dem Kollimator ist.

FIG.1.

FIG.2a.

EP 1 877 831 B1

# FIG.3.

Image Dans Le Plan XY (mm)

## FIG.4.

FIG.5.

# FIG.6a.

# FIG.6b.

FIG.7a.

$y = 0,2858x + 4,8996$

Epaisseur de plexiglass (mm)

LTMH (mm)

♦ 10,2 kBq ▲ 13,8 kBq
× 23,1 kBq ♦ 26,2 kBq
× 35,7 kBq

FIG.7b.

**EP 1 877 831 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2823092
- US 6671541 B
- US 6242744 B
- WO 9637791 A
- US 2003081716 A